# FASCICULE DE BREVET EUROPEEN

(11) **EP 2 774 992 B1**
(45) Date de publication et mention de la délivrance du brevet: **19.12.2018**
(21) Numéro de dépôt: 14305162.1
(22) Date de dépôt: 06.02.2014
(51) Int. Cl.: C12P 7/10, C12P 19/14, C12P 7/14

(54) **Procédé de production d'alcools et/ou de solvants à partir de biomasse lignocellulosique avec lavage du residu solide obtenu après hydrolyse**
Verfahren zur Herstellung von Alkohol und / oder Lösungsmittel aus Biomasse durch Waschen des lignocellulosehaltigen, festen Rückstands der nach der Hydrolyse erhaltet wird
Process for production of alcohol and / or solvents from biomass with washing of the lignocellulosic solid residue obtained after hydrolysis

(30) Priorité: 06.03.2013 FR 1351992
(43) Date de publication de la demande: 10.09.2014
(73) Titulaire: IFP Energies nouvelles, 92500 Rueil-Malmaison (FR)
(72) Inventeur: Aymard, Caroline, 69002 Lyon (FR); Bouillon, Pierre-Antoine, 69003 Lyon (FR); Fleurier, Stéphanie, 69007 Lyon (FR); Louret, Sylvain, 69003 Lyon (FR); Perotta, Larissa, 69007 Lyon (FR); Toth, Eszter, 69008 Lyon (FR)
(74) Mandataire: IFP Energies nouvelles

(56) Documents cités:
- EP-A1- 2 336 343
- WO-A1-83/01627
- WO-A1-2007/147264
- US-A1- 2002 197 686
- Chaogang Liu ET AL: "Continuous Fermentation of Hemicellulose Sugars and Cellulose to Ethanol", , 27 septembre 2005 (2005-09-27), XP055090067, Extrait de l'Internet: URL:http://www.eri.ucr.edu/ISAFXVCD/ISAFXV PP/CtFHSCE.pdf [extrait le 2013-11-25]

## Description

La présente invention s'inscrit dans le cadre d'un procédé de production d'alcools et/ou de solvants dit de "seconde génération" à partir de biomasse lignocellulosique. Elle concerne plus particulièrement un procédé de production d'éthanol et/ou de solvants.

La biomasse lignocellulosique représente une des ressources renouvelables les plus abondantes sur terre. Les substrats considérés sont très variés, puisqu'ils concernent à la fois les substrats ligneux (feuillus et résineux), les sous-produits de l'agriculture (paille) ou ceux des industries génératrices de déchets lignocellulosiques (industries agroalimentaires, papeteries).

La biomasse lignocellulosique est composée de trois principaux polymères : la cellulose (35 à 50%), qui est un polysaccharide essentiellement constitué d'hexoses ; l'hémicellulose (20 à 30%), qui est un polysaccharide essentiellement constitué de pentoses ; et la lignine (15 à 25%), qui est un polymère de structure complexe et de haut poids moléculaire, composé d'alcools aromatiques reliés par des liaisons éther. Ces différentes molécules sont responsables des propriétés intrinsèques de la paroi végétale et s'organisent en un enchevêtrement complexe.

Le procédé de transformation biochimique des matériaux lignocellulosiques en éthanol comprend en général une étape de prétraitement physico-chimique, suivie d'une étape d'hydrolyse enzymatique utilisant un cocktail enzymatique, d'une étape de fermentation éthanolique des sucres libérés et d'une étape de purification des produits de fermentation. Un exemple est donné par le document *"*Ethanol from lignocellulosics: A review of the economy", M. von Silvers et G. Zacchi, Bioresource Technology 56 (1996) 131-140.

Parmi les trois polymères de base qui intègrent la biomasse lignocellulosique, la cellulose et l'hémicellulose sont ceux qui peuvent le plus facilement être valorisés en produits de fermentation. La lignine reste inerte dans la plus grande partie des procédés. C'est pourquoi les procédés de production d'alcools et/ou de solvants ont intérêt à séparer la lignine des mélanges réactionnels au plus tôt, afin de réduire la taille des unités et les coûts de traitement et investissement. La lignine peut être séparée à différentes étapes du procédé, par exemple au prétraitement, entre les étapes d'hydrolyse enzymatique et de fermentation ou dans l'étape de purification des produits de fermentation.

Le document *"*Fuel ethanol production : Process design trends and intégration opportunities", C.A. Cardona et O. J. Sanchez, Bioresource Technology 98 (2007) 2415-2457, décrit un procédé où la lignine est solubilisée en présence d'un solvant permettant de séparer la lignine de la cellulose et de l'hémicellulose, la lignine étant ensuite précipitée pendant le prétraitement de la biomasse. Cette solution évite la présence de solides inertes dans le procédé dès l'étape d'hydrolyse enzymatique, mais présente des coûts opératoires élevés. De plus, cette configuration de procédé ne favorise pas une possible co-fermentation des sucres cellulosiques et hémicellulosiques récupérés au prétraitement, vu que deux flux présentant des propriétés et compositions distinctes sont obtenus.

Certains procédés ("Ethanol from lignocellulosic biomass: technology, economics, and opportunities", C.E. Wyman, Bioresource Technology 50 (1994) 3-16) prévoient la séparation de la lignine seulement en phase de purification des produits issus de l'étape de fermentation. Ceci engendre des investissements supplémentaires, notamment pour les réacteurs de fermentation qui doivent être dimensionnés pour contenir de la lignine inerte en plus du mélange réactionnel et pour la section de séparation qui doit effectuer la séparation entre les produits de fermentation et la lignine, dans le cas où une distillation en présence de solides est envisagée.

La majorité des procédés sépare la lignine des autres produits entre les étapes d'hydrolyse enzymatique et fermentation. Cependant, cette séparation est généralement réalisée par des outils de séparation physique, qui présentent le désavantage que les solides inertes récupérés peuvent contenir encore des produits d'hydrolyse piégés. Dans ce cas, il est possible d'ajouter une étape de lavage des solides au procédé, l'effluent de cette étape de lavage étant envoyé en étape de fermentation avec le flux liquide. Ceci représente un compromis entre la récupération des sucres et les limitations apportées par la pénalité énergétique liée à la dilution du moût à fermenter. Le document *"*A techno-economical comparison of three processes for the production of ethanol from pine", M. von Silvers et G. Zacchi, Bioresource Technology 51 (1995) 46-52, décrit l'impact du lavage du solide récupéré en termes de coût d'équipement de fermentation, ainsi qu'une baisse du titre éthanolique obtenu en fin de fermentation.

Le document US 2002/197686 divulgue un procédé de production de sucre avec lavage des solides obtenus après hydrolyse avec de l'eau mais ne propose pas un lavage de la matière solide avec des vinasses.

Le recyclage des vinasses est bien connu de l'homme du métier, par exemple, le document WO83/01627 divulgue un procédé de production d'alcool et des solvants don une partie des vinasses sont utilisées dans le réacteur d'hydrolyse et une autre partie pour le lavage du solide issue d'un fermenteur qui est ensuite recyclé dans le fermenteur.

La présente invention propose d'effectuer la séparation de la lignine et d'autres éventuels solides inertes entre les étapes d'hydrolyse enzymatique et fermentation. Ce solide composé principalement de lignine est ensuite soumis à un lavage pour récupérer les produits d'hydrolyse piégés, en particulier les sucres. Le liquide de lavage est ensuite recyclé dans l'unité d'hydrolyse enzymatique afin de ne pas apporter de la dilution aux flux existants.

De manière générale, l'invention concerne un procédé de production d'alcool et/ou de solvant à partir d'une charge de biomasse, dans lequel on effectue les étapes suivantes :
a) on effectue une étape de prétraitement par mise en contact et chauffage de la charge de biomasse avec de l'eau et un composé acide ou basique, de manière à obtenir un substrat prétraité,
b) on met en contact le substrat prétraité avec au moins des enzymes cellulases et avec un flux liquide de lavage obtenu à l'étape d) de manière à obtenir un hydrolysat comportant une matière solide et une phase liquide contenant des sucres,
c) on extrait au moins une partie de la matière solide contenue dans l'hydrolysat de manière à obtenir un hydrolysat appauvri en matière solide et un flux enrichi en matière solide,
d) on lave le flux enrichi en matière solide avec un flux liquide comportant au moins une partie du flux de vinasse obtenu à l'étape f) de manière à obtenir ledit flux liquide de lavage, le flux liquide de lavage étant recyclé à l'étape b),
e) on effectue une fermentation alcoolique de l'hydrolysat appauvri en matière solide obtenu à l'étape c) au moyen d'un microorganisme alcooligène de manière à produire un vin de fermentation,
f) on effectue une étape de séparation du vin de fermentation de manière à obtenir au moins un flux purifié comportant un alcool ou un solvant et au moins un flux de vinasse.

A l'étape b), le flux liquide de lavage peut avoir un débit compris entre 50% et 1500% poids du débit de substrat prétraité.

Avant d'effectuer l'étape b), le flux liquide de lavage obtenu à l'étape d) peut être soumis à une deuxième étape de fermentation alcoolique au moyen d'un organisme alcooligène.

On peut effectuer la deuxième étape de fermentation alcoolique dans des conditions opératoires différentes des conditions opératoires de la fermentation alcoolique de l'étape e). On peut également effectuer la deuxième étape de fermentation alcoolique avec un organisme alcooligène différent de l'organisme alcooligène de la fermentation alcoolique de l'étape a).

A l'étape d), on peut mettre en contact le flux enrichi en matière solide avec un flux liquide, puis on peut séparer le flux liquide de la matière solide.

On peut réaliser l'étape d) de manière à ce que ledit flux enrichi en matière solide comporte entre 15% poids et 55% poids de matière solide et de manière à ce que ledit hydrolysat appauvri en matière solide comporte moins de 15% poids de matière solide.

Les enzymes cellulases et/ou hemicellulases peuvent être produites par un microorganisme choisi parmi les champignons appartenant aux genres *Trichoderma, Aspergillus, Penicillium ou Schizophyllum*, ou les bactéries anaérobies appartenant au genre *Clostridium.*

Procédé selon l'une des revendications précédentes, dans lequel le microorganisme alcooligène est choisi parmi les microorganismes du genre *Saccharomyces*, *Saccharomyces cerevisiae, Schizosaccharomyces pombe, Saccharomyces uvarum, Saccharomyces diastaticus, Kluyveromyces fragilis, Candida shehatae, Pichia stipitis, Pachysolen tannophilis , Zymomonas mobilis, Clostridium, Escherichia coli.*

La charge de biomasse peut être composée par au moins l'un des éléments suivants : du bois, de plantes cultivées, des déchets lignocellulosiques agricoles, des résidus de l'industrie de transformation des matériaux lignocellulosiques.

A l'étape a), on peut effectuer une explosion à la vapeur de la biomasse en comprimant, puis en effectuant une détente de la biomasse en mélange avec de l'eau et un composé acide.

A l'étape e) on met en oeuvre un organisme alcooligène produisant au moins de l'éthanol.

D'autres caractéristiques et avantages de l'invention seront mieux compris et apparaîtront clairement à la lecture de la description faite ci-après en se référant aux dessins parmi lesquels :
- la figure 1 est une représentation schématique d'un mode de réalisation non conforme à l'invention,
- la figure 2 est une représentation schématique d'un premier mode de réalisation du procédé suivant l'invention,
- la figure 3 est une représentation schématique d'un deuxième mode de réalisation du procédé suivant l'invention.

Au sens de la présente invention, on désigne sous le terme de "matière sèche" les composés solides et solubles contenus dans un flux, le taux de matière sèche d'un flux est déterminé selon la méthode ASTM E1756-01, qui consiste en une perte de masse à 105°C. On désigne sous le terme "matière solide", les composés solides présents dans un flux, ces composés solides étant non soluble en phase liquide. La matière solide peut être composée de lignine, d'hémicellulose et/ou de cellulose. Le taux de matière solide contenue dans un flux peut être déterminé par lavages successifs du flux à l'eau et analyse de la teneur en matière sèche résiduelle du flux lavé.

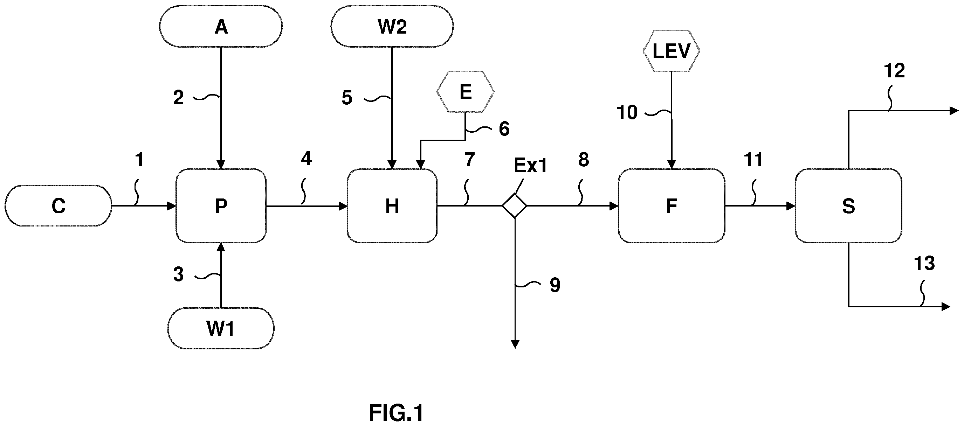

En référence à la figure 1 une charge de biomasse C est amenée dans l'unité de prétraitement P par l'intermédiaire du conduit 1. La charge de biomasse peut être composée de bois, des pailles ou de rafles de maïs, de produits de cultures forestières dédiées (par exemple de résineux tels les épicéas ou les pins, ou des feuillus tels les eucalyptus), des plantes de cultures dédiées tells le miscanthus ou le switchgrass, de résidus de plantes alcooligènes, sucrières (par exemple canne à sucre ou betterave) et céréalières (par exemple mais, blé...), de produits et résidus de l'industrie papetière et des produits de transformation des matériaux lignocellulosiques. La charge peut être composée d'environ 35 à 50% poids de cellulose, de 20 à 30% poids d'hémicellulose et de 15 à 25% poids de lignine.

Le composé acide ou basique A et l'eau W1 nécessaires sont respectivement amenés dans l'unité de prétraitement P par l'intermédiaire des conduits 2 et 3 afin d'y réaliser une réaction d'hydrolyse en milieu acide ou basique. Dans l'unité P, la charge de biomasse C est mise en contact et mélangée avec l'eau W1 et le composé A dans un réacteur. L'unité P de prétraitement peut mettre en oeuvre une action mécanique, créée par exemple au moyen d'une extrudeuse de type bi-vis ou d'une défibreuse.

Parmi les composés acides, le composé A peut être choisi parmi de l'acide sulfurique, de l'acide chlorhydrique, de l'acide nitrique, de l'acide acétique ou de l'acide formique. Parmi les composés basiques, le composé A peut être choisi parmi de l'hydroxyde de potassium, de l'hydroxyde de sodium, de l'ammoniaque.

Lors de l'étape de prétraitement dans l'unité P, on effectue au moins une étape de chauffage du mélange de biomasse C, d'eau W1 et du composé A dans un réacteur. L'eau W1 peut être introduite sous forme de vapeur. Le rôle du prétraitement est de rendre la cellulose accessible aux enzymes en déstructurant la matrice lignocellulosique. Lors du prétraitement, on attaque préférentiellement l'hémicellulose, qui se retrouve en grande partie dissous dans la phase liquide.

Selon un premier mode de réalisation, on réalise un prétraitement alcalin dans l'unité P. Par exemple, dans l'unité P, on peut mettre en oeuvre un prétraitement au sulfate de sodium, encore appelé procédé Kraft, classiquement utilisé dans les procédés de production de produits papetiers, dit Kraft ou "pâte au sulfate", à l'issue duquel on obtient des pâtes papetières. Le prétraitement chimique alcalin réalisé dans l'unité P peut également être un prétraitement par explosion des fibres à l'ammoniac, encore appelé prétraitement AFEX (Ammonia Fiber Explosion) ou prétraitement par percolation utilisant de l'ammoniaque avec recycle, encore appelé prétraitement ARP (Ammonia Recycle Percolation).

Le procédé au sulfate de sodium ou procédé Kraft est basé sur l'utilisation de soude et de sulfate de sodium. Le traitement chimique des copeaux de bois se fait à 150-175°C pendant une durée de 1 à 7 heures en fonction du substrat utilisé. Les pâtes papetières kraft sont produites à partir des biomasses les plus variées, mais plus particulièrement depuis les espèces arborescentes résineuses (softwood tels que les épicéas ou les pins) ou feuillues (hardwood tels que les eucalyptus) ou encore les déchets lignocellulosiques agricoles (paille de blé, riz, etc.). Elles sont partiellement délignifiées au moyen de cuissons à haute température et en présence de soude. Cette délignification est contrôlée par les paramètres opératoires des réacteurs. La cuisson est réalisée dans un réacteur vertical, où les copeaux descendent par gravité et rencontrent les diverses liqueurs de cuisson. Le sulfure de sodium est préparé directement à partir de sulfate de sodium par combustion. Lors de la cuisson, le sulfure de sodium est hydrolysé en soude, en NaHS et en H₂S. Les différents composés soufrés présents réagissent avec la lignine pour donner des thiolignines plus facilement solubles. La liqueur appliquée aux copeaux est appelée liqueur blanche. La liqueur extraite du réacteur ou lessiveur contenant les composés éliminés de la paroi est appelée liqueur noire. A l'issue de ce prétraitement alcalin, on aboutit à la production d'un substrat prétraité, enrichi en cellulose puisqu'il contient entre 60 et 90% de cellulose et entre 5 et 20% d'hémicellulose.

Le procédé ARP (Ammonia Recycle Percolation) est un procédé de prétraitement utilisant de l'ammoniaque avec recyclage. Ce type de procédé est notamment décrit par Kim et al., 2003, Biores. Technol. 90 (2003) p 39-47. La température élevée de la percolation conduit à une solubilisation partielle à la fois de la lignine et des hémicelluloses, cette solution est ensuite chauffée pour recycler l'ammoniaque et récupérer d'une part la lignine extraite, par exemple pour une valorisation énergétique, et d'autre part les sucres solubles issus des hémicelluloses.

Le procédé AFEX (Ammonia Fiber Explosion) consiste à introduire le substrat lignocellulosique dans un cuiseur à haute pression en présence d'ammoniac, puis de provoquer une détente explosive en sortie du réacteur et de recycler l'ammoniac alors sous forme gazeuse. Ce type de procédé est notamment décrit par Teymouri et al., 2005, Biores. Technol. 96 (2005) p.2014-2018. Ce procédé conduit principalement à une déstructuration de la matrice de la biomasse mais il n'y a pas de séparation phasique des composés lignine, hémicellulose et cellulose en sortie de traitement.

Selon un deuxième mode de réalisation, on réalise un prétraitement acide dans l'unité P. Par exemple, dans l'unité P, on peut mettre en oeuvre un prétraitement de type cuisson à l'acide diluée. Dans ce mode de réalisation la biomasse est mise en contact avec un acide fort dilué dans de l'eau, par exemple l'acide sulfurique, en mettant en oeuvre la biomasse à de faibles teneurs en matières sèche, généralement compris entre 5 et 20% de matière sèche. La biomasse, l'acide et l'eau sont mis en contact dans un réacteur et monté en température, généralement entre 120°C et 200°C. Lors de ce procédé, les composés hémicellulosique sont principalement hydrolysés en sucres, permettant de déstructurer la matrice lignocellulosique. A l'issue de ce prétraitement acide, on aboutit à la production d'un substrat prétraité solide, enrichi en cellulose et en lignine ainsi qu'une fraction liquide enrichie en sucres.

Selon un troisième mode de réalisation on peut également mettre en oeuvre le procédé nommé "explosion vapeur", ou "SteamEx" ou "Steam Explosion" selon la terminologie anglo-saxonne, dans l'unité P. C'est un procédé dans lequel la biomasse lignocellulosique est mise en contact avec de l'eau dans un réacteur à faible temps de séjour, généralement compris entre 2 et 15 minutes et à des températures modérées, généralement entre 120°C et 250°C et à une pression comprise entre 5 et 50 bars. L'eau peut être additionné d'un composé acide, par exemple de l'acide sulfurique, ou d'un composé basique. En sortie de du réacteur, la biomasse est détendue, par exemple à pression atmosphérique, dans un récipient séparateur gaz / solide afin de produire une biomasse prétraitée à haute matière sèche, généralement comprise entre 20 et 70% de matière sèche.

L'unité P peut comporter des étapes additionnelles, par exemple de mise au pH, qui ont pour but de faciliter la mise en oeuvre et l'efficacité des étapes d'hydrolyse enzymatique et de fermentation.

On évacue de l'unité P un substrat prétraité par le conduit 4. Le substrat prétraité est composé de sucres dissous en phase liquide et de matière solide composée de lignine, de cellulose et d'hémicellulose qui n'a pas été liquéfié dans le prétraitement P. Le flux de substrat prétraité circulant dans le conduit 4 contient préférentiellement entre 10% poids et 60% poids de matière sèche et encore plus préférentiellement entre 20% poids et 55% poids de matière sèche.

Le substrat prétraité est introduit dans un réacteur de l'unité H pour subir une étape dite d"hydrolyse enzymatique". De l'eau W2 et des enzymes E sont respectivement ajoutés dans le réacteur l'unité H par l'intermédiaire des conduits 5 et 6 afin de réaliser une réaction d'hydrolyse enzymatique du substrat prétraité. Les quantités de substrat prétraité d'eau et d'enzyme sont ajustées dans l'étape dans l'unité H de manière à ce que le milieu réactionnel comporte une teneur en matière solide généralement comprise entre 5% et 40% poids, de préférence entre 10% et 25% poids. L'hydrolyse enzymatique est préférentiellement réalisée à pH compris entre 4 et 5,5 et à une température comprise entre 35°C et 60°C. Les enzymes E peuvent être produites par un microorganisme, par exemples des champignons appartenant aux genres *Trichoderma, Aspergillus, Penicillium ou Schizophyllum,* ou les bactéries anaérobies appartenant par exemple au genre *Clostridium.* Les enzymes produites par ces microorganismes contiennent notamment les cellulases et éventuellement des hémicellulases, adaptées à réaliser une hydrolyse poussée de la cellulose et éventuellement des hémicelluloses. Les cellulases, respectivement les hémicelluloses transforment par hydrolyse la cellulose, respectivement l'hémicellulose, en sucres qui peuvent se dissoudre en phase aqueuse. Dans l'unité H, les conditions de l'hydrolyse enzymatique, principalement le taux de matière sèche du mélange à hydrolyser et la quantité d'enzymes utilisée, sont choisies de telle façon que l'on obtienne une solubilisation de la cellulose comprise entre 20% et 99% poids, de préférence entre 30% et 95% poids par rapport au poids total de cellulose contenu dans le substrat prétraité. On évacue de l'unité H un substrat hydrolysé par le conduit 7. Ainsi le flux de substrat hydrolysé issu de H comporte des sucres dissous en phase aqueuse et de la matière solide composée principalement de lignine, et de cellulose et d'hémicellulose qui n'ont pas été hydrolysées.

Ledit substrat hydrolysé subit ensuite, dans l'unité Ex1, une étape de séparation entre liquide et solide afin d'en extraire la matière solide, notamment la lignine qui n'a pas été hydrolysée dans l'unité H. L'extraction de la matière solide est effectuée dans l'unité Ex1, qui peut mettre en oeuvre l'une des techniques suivantes : centrifugation, essorage ou pressage, filtration, décantation. L'unité Ex1 produit un flux appauvri en matière solide évacué par le conduit 8 et un flux enrichi en matière solide, notamment en lignine, évacué par le conduit 9.

Le flux appauvri en solide est ensuite introduit par le conduit 8 dans l'unité F pour subir une étape de fermentation. Dans l'unité F, le flux appauvri en solide est mis en contact avec un ou plusieurs micro-organismes de fermentation LEV introduits par le conduit 10. Les microorganismes LEV peuvent être choisis par exemple parmi les éléments suivants : les levures du genre *Saccharomyces, Saccharomyces cerevisiae, Schizosaccharomyces pombe, Saccharomyces uvarum, Saccharomyces diastaticus, Kluyveromyces fragilis, Candida shehatae, Pichia stipitis, Pachysolen tannophilis* ou les bactéries du genre *Zymomonas mobilis, Clostridium, Escherichia coli. De préférence on utilise une levure adaptée à produire de l'éthanol, par exemples les levures* ... Les sucres fermentescibles sont ainsi transformés en alcools et/ou solvants par les micro-organismes. L'étape de fermentation dans l'unité F peut être réalisée à une température comprise entre 30°C et 35°C. Dans cette unité F, la réaction de fermentatbn produit un vin de fermentation contenant les produits de la réaction de fermentation, évacué par le conduit 11, par exemple des alcools ou des solvants organiques.

Le vin de fermentation est introduit par le conduit 11 dans l'unité de séparation S afin d'en extraire les composés d'intérêt évacués par le conduit 12, par exemple des alcools ou des solvants organiques. Les résidus de la séparation, couramment appelés vinasses, sont évacués de l'unité de séparation S par le conduit 13. Les vinasses sont généralement composées d'eau ainsi que de tout produit liquide ou solide non converti ou non extrait lors des étapes précédentes dans les unités H, Ex1 et F. L'unité de séparation S peut mettre en oeuvre une ou plusieurs distillations, et éventuellement une séparation des matières en suspension par exemple par centrifugation, décantation, filtration.

Le procédé non conforme à l'invention schématisé par la figure 1 présente l'inconvénient d'évacuer une partie des composés valorisables, c'est-à-dire des sucres, qui restent contenus dans la matière solide évacuée par le conduit 9 lors de l'opération d'extraction des solides dans l'unité Ex1. Ces sucres présents sous forme liquide dans l'hydrolysat circulant dans le conduit 7 risquent d'être séparés de façon imparfaite dans l'étape Ex1 avec les outils connus de l'homme de l'art, par exemple des outils de centrifugation, essorage, décantation ou pressage. Au moins une fraction des sucres est évacuée par le conduit 9 provoquant une perte de rendement du procédé.

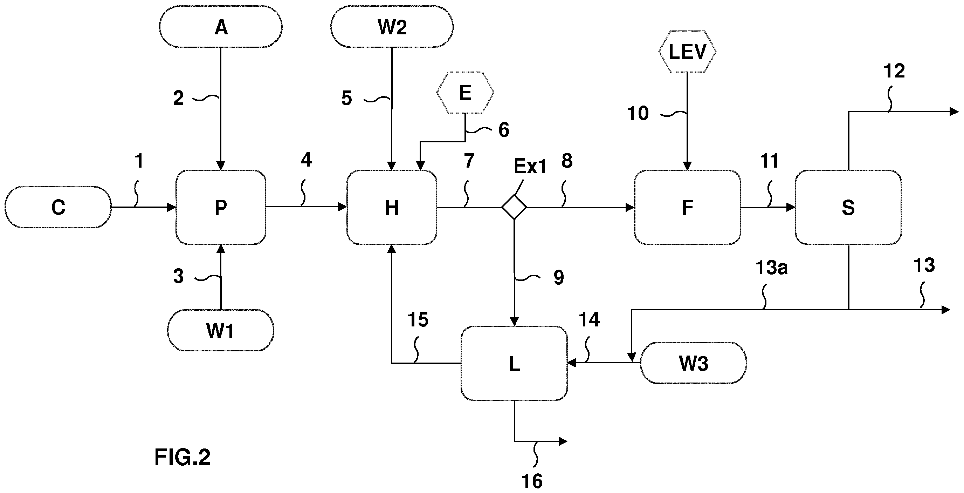

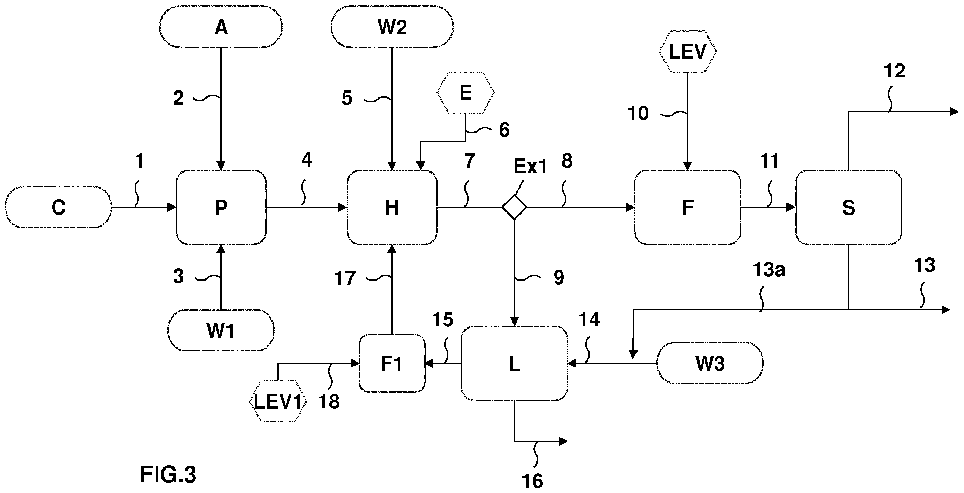

Le procédé selon l'invention propose de remédier à ce problème de perte de sucres dans la matière solide en mettant en oeuvre une opération de lavage du flux enrichi en solide circulant dans le conduit 9, permettant de recycler les sucres dans le procédé sans y apporter une dilution supplémentaire. L'invention sera mieux comprise à la lecture de la description des figures 2 et 3, schématisant deux mises en oeuvre du procédé selon l'invention. Les références de la figure 2 identiques à celles de la figure 1 désignent les mêmes éléments.

Selon l'invention, on peut effectuer l'étape de séparation de la matière solide du liquide dans l'unité Ex1 de manière à ce que le flux 8 appauvri en solide contienne moins de 15% poids, et de préférence moins de 10% poids, et encore plus préférentiellement moins de 5% poids de matières solide. Le reste du flux 8 peut être composé de sucre dissous en phase aqueuse. De plus, on peut effectuer l'étape de séparation de la matière solide du liquide dans l'unité Ex1 de manière à ce que le flux 9 enrichi en solides contienne entre 15% et 55% poids, et de préférence entre 20% et 45% poids et encore plus préférentiellement entre 25% et 35% poids de matière solide. Du fait des limitations des équipements de séparation entre solides et liquides de l'unité Ex1, le flux 9 enrichi en solides contient au moins 45% de liquide, qui peut notamment être composé de sucre dissous en phase aqueuse.

En référence à la figure 2, on effectue, dans l'unité L, une étape de lavage de la matière solide contenue dans le flux arrivant par le conduit 9. Dans l'unité L, un flux liquide est amené par l'intermédiaire du conduit 14 afin de réaliser un lavage de la matière solide contenue dans le flux arrivant par le conduit 9. Le flux liquide est mis en contact avec la matière solide, puis le liquide est séparé de la matière solide. L'étape de lavage dans l'unité L peut être réalisée par percolation, par opérations de mélange et séparation liquide/solide successives ou par toute autre technique connue de l'homme de l'art. Le lavage permet d'extraire par le conduit 15 un flux liquide enrichi en composés d'intérêt, c'est-à-dire des sucres, ainsi qu'un flux appauvri en composés d'intérêt par le conduit 16. Le flux 15 est ensuite recyclé dans l'unité H, afin de réutiliser les sucres dans l'étape d'hydrolyse enzymatique.

En référence à la figure 2, le flux liquide amené par le conduit 14 peut être un flux d'eau fraiche W3 ou une portion des vinasses provenant de l'unité S amenée par les conduits 13 puis 13a dans l'unité L. Selon l'invention, le fait de recycler le flux enrichi en composés d'intérêt par le conduit 15 dans l'unité H permet de limiter, voire de supprimer, l'apport d'eau fraiche W2 directement dans l'unité H. Par exemple le flux 15 représente entre 50%poids et 1500%poids, préférentiellement entre 100%poids et 600%poids, du débit de substrat prétraité introduit par le conduit 4 dans l'unité H. Ainsi, la présente invention permet de limiter, voire d'éviter, toute dilution supplémentaire des flux dans le procédé liée à l'emploi d'eau pour le lavage du flux 9.

Le flux circulant dans le conduit 15 contient des composés hydrolysés valorisables, notamment des sucres, qui n'ont pas été envoyés en fermentation. Le recyclage de ces composés dans l'unité H d'hydrolyse enzymatique peut avoir un effet inhibiteur sur cette étape du procédé, dû à l'accumulation des produits de réaction dans le milieu. Alternativement, ces composés peuvent nécessiter des conditions de fermentation et/ou des microorganismes de fermentation qui sont différents des ceux de l'étape F. Afin de réduire l'impact négatif du recyclage sur le rendement de l'unité H ou pour augmenter la production de produits issus de la fermentation, la présente invention propose la mise en oeuvre du procédé de la figure 3 dans laquelle le flux circulant dans le conduit 15 est dirigé vers une étape de fermentation supplémentaire avant d'être renvoyé à l'unité H. Les références de la figure 3 identiques à celles des figures 1 et 2 désignent les mêmes éléments.

En référence à la figure 3, le flux 15 issu de l'unité de lavage L est mis en contact dans l'unité F1 avec des microorganismes LEV1 introduits par le conduit 18. Les microorganismes LEV1 peuvent être choisis par exemple parmi les éléments suivants : les levures du genre *Saccharomyces, Saccharomyces cerevisiae, Schizosaccharomyces pombe, Saccharomyces uvarum, Saccharomyces diastaticus, Kluyveromyces fragilis, Candida shehatae, Pichia stipitis, Pachysolen tannophilis* ou les bactéries du genre *Zymomonas mobilis, Clostridium, Escherichia coli.* Dans l'unité F1, une réaction de fermentation a lieu, produisant un vin de fermentation enrichi en produits de la réaction de fermentation, par exemple des alcools ou des solvants organiques, évacué par le conduit 17. Ce vin, appauvri en produits hydrolysés, mais riche en composés valorisés, est recyclé dans l'unité H par le conduit 17. Son recycle permet de réduire la dilution des flux traités, tout en valorisant les composés d'intérêt récupérés, notamment les sucres transformés en produits de fermentation, et en assurant une augmentation du rendement global du procédé.

L'étape de fermentation dans l'unité F1 peut être identique ou différente à la fermentation réalisée dans l'unité F, selon les besoins du procédé. Le fait de mettre en oeuvre une fermentation dans l'unité F1 différente de celle réalisée de l'unité F permet de traiter sous des conditions de fermentation spécifique des composés qui ne sont pas ou peu fermenté dans l'unité F. Par exemple l'unité F est opérée à une température différente de celle de l'unité F1, ou bien l'unité F met en oeuvre un microorganisme (par exemple S. *cerevisiae*) différent de celui mis en oeuvre dans l'unité F1 (par exemple *C.shehatae*), afin de fermenter de manière optimisée différents composés d'intérêt. Le mode de réalisation de la figure 3 est particulièrement bien adapté au cas où au moins une partie des vinasses du flux 13 sont utilisées comme liquide de lavage dans l'unité L. En effet, la composition du flux 15 dans ce cas est très différente de celle du flux 8 et des conditions opératoires et/ou d'autres levures peuvent être mieux adaptées au milieu.

### EXEMPLES

Les exemples ci-après illustrent l'invention.

### Exemple 1 selon le procédé de la figure 1 (non conforme à l'invention)

Dans cet exemple, on présente un procédé de production d'éthanol à partir de paille avec les caractéristiques suivantes:
Charge: Paille de blé, débit 89,60 tonne / heure, composition moyenne :

| | % (base sèche) |
|---|---|
| Cellulose | 39,1 % |
| Hémicellulose | 26,9% |
| Lignine | 17,9% |
| Autres (cendres, extractibles, etc...) | 16,1% |

### Préparation de charge et prétraitement dans l'unité P :

La paille est broyée sur une grille 50mm, puis imprégnée avec l'acide H₂SO₄ dilué à 0,7 g/litre. L'imprégnation est suivie d'une séparation solide/liquide et la liqueur d'imprégnation est recyclée, la matière sèche (MS) du solide en entrée de prétraitement est de 45%. Le prétraitement par explosion à la vapeur est effectué à 200°C dans une configuration continue employant un court temps de séjour. Le milieu est brusquement détendu à une pression de 1,3 atm. Le substrat prétraité est envoyé vers le réacteur d'hydrolyse enzymatique.

### Hydrolyse enzymatique et séparation :

La concentration en solides à l'entrée du réacteur d'hydrolyse enzymatique dans l'unité H est de 14%. Pour atteindre ce niveau de dilution, 114,3 tonnes / heure d'eau W2 de procédé sont mélangées au substrat prétraité. Dans les conditions de mise en oeuvre de l'hydrolyse enzymatique, un moût contenant 85,5 g d'hexoses par kg de moût est produit. Ce moût est envoyé vers une décanteuse dans l'unité Ex1, où la matière solide, notamment la lignine, est séparée du moût avant l'étape de fermentation des sucres produits. Dans cette étape de séparation, le liquide clarifié est envoyé en fermentation et un flux contenant 28,4% de matière solide est produit. Étant donnée la nature de la séparation, ce flux contient encore une quantité importante de sucres piégés. La perte de sucres lors de cette étape est estimée à 20,7%.

### Conversion en éthanol :

Le liquide clarifié est envoyé vers les fermenteurs de l'unité F. Le microorganisme de fermentation est *Saccharomyces cerevisiae.* Couplé aux rendements de l'hydrolyse enzymatique, le rendement global de la conversion de la cellulose en éthanol est de 0,41 g d'éthanol par g de cellulose introduite. Les pentoses présents ne sont pas convertis par le microorganisme de fermentation choisi. Le titre éthanolique du flux envoyé en distillation est de 43,9 g d'éthanol / kg de vin.

### Séparation et récupération de l'éthanol :

La séparation dans l'unité S se fait par distillation. Le rendement d'extraction est de 99,6%. Ainsi, le procédé permet de produire annuellement **76 889 tonnes d'éthanol,** et a une consommation spécifique d'eau de procédé de **11,9 tonnes d'eau / tonne d'éthanol produit.**

### Exemple 2 selon le procédé de la figure 1 (non conforme à l'invention)

Dans cet exemple, on présente un procédé de production d'éthanol à partir de paille avec les caractéristiques suivantes:
Charge: Paille de blé, débit 89,60 tonne / heure, composition moyenne :.

| | % (base MS) |
|---|---|
| Cellulose | 39,1 % |
| Hémicellulose | 26,9% |
| Lignine | 17,9% |
| Autres (cendres, extractibles, etc...) | 16,1% |

### Préparation de charge et prétraitement dans l'unité P :

La paille est broyée sur une grille 50mm, puis imprégnée avec l'acide H₂SO₄ dilué à 0,7 g/litre. L'imprégnation est suivie d'une séparation solide/liquide et la liqueur d'imprégnation est recyclée, la MS du solide en entrée de prétraitement est de 45%. Le prétraitement par explosion à la vapeur est effectué à 200°C dans une configuration continue employant un court temps de séjour. Le milieu est brusquement détendu à une pression de 1,3 atm. Le substrat prétraité est envoyé vers le réacteur d'hydrolyse enzymatique dans l'unité H.

### Hydrolyse enzymatique et séparation :

La concentration en solides à l'entrée du réacteur d'hydrolyse enzymatique dans l'unité H est de 14%. Pour atteindre ce niveau de dilution, 114,3 tonnes / heure d'eau de procédé sont mélangées au substrat prétraité. Dans les conditions de mise en oeuvre de l'hydrolyse enzymatique, un moût contenant 85,5 g d'hexoses par kg de moût est produit. Ce moût est envoyé vers une décanteuse dans l'unité Ex1, où la matière solide, notamment la lignine, est séparée du moût avant l'étape de fermentation des sucres produits. Dans cette étape de séparation, le liquide clarifié est envoyé en fermentation et un flux contenant 28,4% de matière solide est produit.

### Lavage du flux de matière solide :

Étant donnée la nature de la séparation, environ 20% des sucres hydrolysés restent piégés dans la matière solide. Un lavage en contrecourant de la matière solide est alors mis en oeuvre dans le but de réduire les pertes. 115 tonnes / heure d'eau de procédé sont utilisées dans cette opération. Les pertes de sucres baissent à **1,4%.** Le jus sucré récupéré est mélangé au liquide clarifié avant d'être envoyé en fermentation.

### Conversion en éthanol :

Le liquide clarifié mélangé au jus sucré est envoyé vers les fermenteurs de l'unité F. Dû à la basse concentration en hexoses des jus de lavage récupérés, le moût ne contient que 76,2 g d'hexoses par kg de moût en entrée de l'étape de fermentation, **valeur 10,9% inférieure à celle obtenu en fin d'hydrolyse enzymatique.**
Le microorganisme de fermentation est *Saccharomyces cerevisiae.* Couplé aux rendements de l'hydrolyse enzymatique, le rendement global de la conversion de la cellulose en éthanol est de 0,50 g d'éthanol par g de cellulose introduite. Les pentoses présents ne sont pas convertis par le microorganisme de fermentation choisi. Le titre éthanolique du flux envoyé en distillation est de 36,0 g d'éthanol / kg de vin.

### Séparation et récupération de l'éthanol :

La séparation dans l'unité S se fait par distillation. Le rendement d'extraction est de 99,6%. Ainsi, le procédé permet de produire annuellement **95 752 tonnes d'éthanol,** et a une consommation spécifique d'eau de procédé de **19,1 tonnes d'eau / tonne d'éthanol produit.**

### Exemple 3 selon le procédé de la figure 2 (conforme à l'invention)

Dans cet exemple, on présente un procédé de production d'éthanol à partir de paille avec les caractéristiques suivantes :
Charge : Paille de blé, débit 89,60 tonne / heure, composition moyenne :

| | % (base MS) |
|---|---|
| Cellulose | 39,1 % |
| Hémicellulose | 26,9% |
| Lignine | 17,9% |
| Autres (cendres, extractibles, etc...) | 16,1% |

### Préparation de charge et prétraitement dans l'unité P :

La paille est broyée sur une grille 50mm, puis imprégnée avec l'acide H₂SO₄ dilué à 0,7 g/litre. L'imprégnation est suivie d'une séparation solide/liquide et la liqueur d'imprégnation est recyclée, la MS du solide en entrée d'explosion est de 45%. Le prétraitement par explosion à la vapeur est effectué à 202°C dans une configuration continue employant un court temps de séjour. Le milieu est brusquement détendu à une pression de 1,3 atm. Le substrat prétraité est envoyé vers le réacteur d'hydrolyse enzymatique.

### Hydrolyse enzymatique et séparation :

La concentration en solides à l'entrée du réacteur d'hydrolyse enzymatique dans l'unité H est de 14%. Pour atteindre ce niveau de dilution, 114,3 tonnes / heure d'eau de dilution arrivant par le conduit 15 sont mélangées au substrat prétraité. Cette eau de dilution contient 51,1 g de produits hydrolysés / kg de solution originaires du recyclage de l'eau utilisée pour le lavage du flux de matière solide obtenu à l'issue de l'opération de séparation liquide/solide en sortie de l'hydrolyse enzymatique. Dans les conditions de mise en oeuvre de l'hydrolyse enzymatique, un moût contenant 97,8 g d'hexoses par kg de moût est produit. Ce moût est envoyé vers une décanteuse dans l'unité Ex1, où la matière solide, notamment la lignine, est séparée du moût avant l'étape de fermentation des sucres produits. Dans cette étape de séparation, le liquide clarifié est envoyé en fermentation et un flux contenant 30,0% de matière solide est produit.

### Lavage du flux de matière solide :

Afin de récupérer les sucres piégés dans la matière solide, une étape de lavage de la matière solide est mise en oeuvre. Dans cet exemple, la totalité de l'eau envoyée comme eau de dilution à l'étape d'hydrolyse enzymatique est d'abord utilisée pour laver la matière solide dans l'unité de lavage L à contrecourant sous trois étages de contact. Puis l'eau de lavage est introduite dans l'unité H. Les pertes de sucres sont alors **réduites de 92,0%** par rapport aux résultats de l'exemple 1, soit **1,6%** de perte globale.

### Conversion en éthanol :

Le liquide clarifié est envoyé vers les fermenteurs de l'unité F. Le microorganisme de fermentation est *Saccharomyces cerevisiae.* Couplé aux rendements de l'hydrolyse enzymatique, le rendement global de la conversion de la cellulose en éthanol est de 0,48 g d'éthanol par g de cellulose introduite. La baisse de rendement s'explique par la plus haute concentration de produits hydrolysés dans l'étape d'hydrolyse enzymatique, qui impacte légèrement la réaction. Les pentoses présents ne sont pas convertis par le microorganisme de fermentation choisi. Le titre éthanolique du flux envoyé en distillation est de 50,7 g d'éthanol / kg de vin.

### Séparation et récupération de l'éthanol :

La séparation dans l'unité S se fait par distillation. Le rendement d'extraction est de 99,6%. Ainsi, le procédé permet de produire annuellement **88 182 tonnes d'éthanol,** et a une consommation spécifique d'eau de procédé de **10,4 tonnes d'eau / tonne d'éthanol produit,** soit 12,6% et 45,5% de réduction par rapport aux consommations d'eau dans les exemples 1 et 2, respectivement.

### Exemple 4 selon le procédé de la figure 3 (conforme à l'invention)

Dans cet exemple, on présente un procédé de production d'éthanol à partir de paille avec les caractéristiques suivantes :
Charge : Paille de blé, débit 89,60 tonne / heure, composition moyenne :

| | % (base MS) |
|---|---|
| Cellulose | 39,1 % |
| Hémicellulose | 26,9% |
| Lignine | 17,9% |
| Autres (cendres, extractibles, etc...) | 16,1% |

### Préparation de charge et prétraitement dans l'unité P :

La paille est broyée sur une grille 50mm, puis imprégnée avec l'acide H₂SO₄ dilué à 0,7 g/litre. L'imprégnation est suivie d'une séparation solide/liquide et la liqueur d'imprégnation est recyclée, la MS du solide en entrée de prétraitement est de 45%. Le prétraitement par explosion à la vapeur est effectué à 200°C dans une configuration continue employant un court temps de séjour. Le milieu est brusquement détendu à une pression de 1,3 atm. Le substrat prétraité est envoyé vers le réacteur d'hydrolyse enzymatique.

### Hydrolyse enzymatique et séparation :

La concentration en solides à l'entrée du réacteur d'hydrolyse enzymatique dans l'unité H est de 14%. Pour atteindre ce niveau de dilution, 114,3 tonnes / heure d'eau de dilution arrivant par le conduit 17 sont mélangées au substrat prétraité. Cette eau de dilution contient 25,6 g d'éthanol / kg de solution, originaire de la fermentation du recycle d'eau de lavage du flux de matière solide issue de l'opération de séparation liquide/solide en sortie de l'hydrolyse enzymatique. Dans les conditions de mise en oeuvre de l'hydrolyse enzymatique, un moût contenant 85,8 g d'hexoses par kg de moût est produit. Ce moût est envoyé vers une décanteuse dans l'unité Ex1, où la matière solide est séparée du moût avant l'étape de fermentation des sucres produits. Dans cette étape de séparation, le liquide clarifié est envoyé en fermentation dans l'unité F et un flux contenant 29,3% de matière solide est produit.

### Lavage du flux de matière solide :

Afin de récupérer les sucres piégés dans la matière solide, une étape de lavage de la matière solide est mise en oeuvre. Dans cet exemple, la totalité de l'eau envoyée comme eau de dilution à l'étape d'hydrolyse enzymatique est d'abord utilisée pour laver la matière solide dans l'unité de lavage L à contrecourant sous trois étages de contact. Les pertes de sucres sont alors **réduites de 93,0%** par rapport aux résultats de l'exemple 1, soit **1,4%** de perte globale.

### Conversion en éthanol :

Le liquide clarifié issu de la décanteuse est envoyé vers les fermenteurs de l'unité F. L'eau de lavage des solides est aussi envoyée vers la fermentation vers les fermenteurs de l'unité F1. Ces deux étapes sont mises en oeuvre séparément. Dans cet exemple, le microorganisme de fermentation est *Saccharomyces cerevisiae* dans les deux ateliers F et F1. Puis l'eau de lavage fermentée dans l'unité F1 est ensuite introduite dans l'unité H. Le rendement global du procédé de conversion de la cellulose introduite en éthanol, comprenant les deux fermentations distinctes et le rendement de l'hydrolyse enzymatique, est de 0,51 g d'éthanol par g de cellulose introduite. L'introduction d'une étape de fermentation de l'eau de dilution avant introduction en hydrolyse enzymatique a un effet positif sur le procédé par rapport à l'exemple 2, car dans ce cas le rendement de l'hydrolyse enzymatique n'est pas impacté par l'eau de dilution recyclée. Les pentoses présents ne sont pas convertis par le microorganisme de fermentation choisi. Le titre éthanolique du flux envoyé en distillation est de 54,6 g d'éthanol / kg de vin.

### Séparation et récupération de l'éthanol :

La séparation se fait par distillation dans l'unité S. Le rendement d'extraction est de 99,6%. Ainsi, le procédé permet de produire annuellement **95 187 tonnes d'éthanol,** et a une consommation spécifique d'eau de procédé de **9,6 tonnes d'eau** / **tonne d'éthanol produit,** soit 19,3% et 49,7% de réduction par rapport aux consommations des exemples 1 et 2, respectivement.

### Exemple 5 selon le procédé de la figure 1 (non conforme à l'invention)

Dans cet exemple, on présente un procédé de production d'éthanol à partir de paille avec les caractéristiques suivantes :
Charge : Paille de blé, débit 89,60 tonne / heure, composition moyenne :

| | % (base MS) |
|---|---|
| Cellulose | 39,1 % |
| Hémicellulose | 26,9% |
| Lignine | 17,9% |
| Autres (cendres, extractibles, etc...) | 16,1% |

### Préparation de charge et prétraitement dans l'unité P :

La paille est broyée sur une grille 50mm, puis imprégnée avec l'acide H₂SO₄ dilué à 0,7 g/litre. L'imprégnation est suivie d'une séparation solide/liquide et la liqueur d'imprégnation est recyclée, la MS du solide en entrée de prétraitement est de 45%. Le prétraitement par explosion à la vapeur est effectué à 200°C dans une configuration continue employant un court temps de séjour. Le milieu est brusquement détendu à une pression de 1,3 atm. Le substrat prétraité est envoyé vers le réacteur d'hydrolyse enzymatique.

### Hydrolyse enzymatique et séparation :

La concentration en solides à l'entrée du réacteur d'hydrolyse enzymatique dans l'unité H est de 14%. Pour atteindre ce niveau de dilution, 114,3 tonnes / heure d'eau de procédé sont mélangées au substrat prétraité. Dans les conditions de mise en oeuvre de l'hydrolyse enzymatique, un moût contenant 85,5 g d'hexoses par kg de moût et 70,0 g de pentoses par kg de moût est produit. Ce moût est envoyé vers une décanteuse dans l'unité Ex1, où la matière solide, notamment la lignine, est séparée du moût avant l'étape de fermentation des sucres produits. Dans cette étape de séparation, le liquide clarifié est envoyé en fermentation et un flux contenant 28,4% de matière solide est produit. Étant donnée la nature de la séparation, ce flux contient encore une quantité importante de sucres piégés. La perte globale de sucres lors de cette étape est estimée à **20,7%.**

### Conversion en éthanol :

Le liquide clarifié est envoyé vers les fermenteurs de l'unité F. Le microorganisme de fermentation est *Saccharomyces cerevisiae* provenant d'une souche modifiée capable de réaliser la co-fermentation des hexoses et des pentoses. Couplé aux rendements de l'hydrolyse enzymatique, le rendement global de la conversion de la cellulose en éthanol est de 0,41 g d'éthanol par g de cellulose introduite. La conversion des pentoses en éthanol s'élève à 0,09 g d'éthanol par g de pentose introduite. Le titre éthanolique du flux envoyé en distillation est de 52,8 g d'éthanol / kg de vin.

### Séparation et récupération de l'éthanol :

La séparation dans l'unité S se fait par distillation. Le rendement d'extraction est de 99,6%. Ainsi, le procédé permet de produire annuellement **91 699 tonnes d'éthanol,** et a une consommation spécifique d'eau de procédé de **10,0 tonnes d'eau / tonne d'éthanol produit.**

### Exemple 6 selon le procédé de la figure 2 (conforme à l'invention)

Dans cet exemple, on présente un procédé de production d'éthanol à partir de paille avec les caractéristiques suivantes :
Charge : Paille de blé, débit 89,60 tonne / heure, composition moyenne :

| | % (base MS) |
|---|---|
| Cellulose | 39,1 % |
| Hémicellulose | 26,9% |
| Lignine | 17,9% |
| Autres (cendres, extractibles, etc...) | 16,1% |

### Préparation de charge et prétraitement dans l'unité P:

La paille est broyée sur une grille 50mm, puis imprégnée avec l'acide H₂SO₄ dilué à 0,7 g/litre. L'imprégnation est suivie d'une séparation solide/liquide et la liqueur d'imprégnation est recyclée, la MS du solide en entrée de prétraitement est de 45%. Le prétraitement par explosion à la vapeur est effectué à 200°C dans une configuration continue employant un court temps de séjour. Le milieu est brusquement détendu à une pression de 1,3 atm. Le substrat prétraité est envoyé vers le réacteur d'hydrolyse enzymatique.

### Hydrolyse enzymatique et séparation :

La concentration en solides à l'entrée du réacteur d'hydrolyse enzymatique dans l'unité H est de 14%. Pour atteindre ce niveau de dilution, 114,3 tonnes / heure d'eau de dilution sont mélangées au substrat prétraité. Cette eau de dilution contient 51,3 g d'hexoses / kg de solution et 121,1 g de pentoses / kg de solution originaires du recyclage des vinasses produites en distillation et utilisées pour le lavage du flux de matière solide obtenu à l'issue de l'opération de séparation liquide/solide en sortie de l'hydrolyse enzymatique. Dans les conditions de mise en oeuvre de l'hydrolyse enzymatique, un moût contenant 98,0 g d'hexoses par kg de moût et 114,4 g de pentoses par kg de moût est produit. Ce moût est envoyé vers une décanteuse dans l'unité Ex1, où la matière solide notamment la lignine est séparée du moût avant l'étape de fermentation des sucres produits. Dans cette étape de séparation, le liquide clarifié est envoyé en fermentation dans l'unité F et un flux contenant 31,4% de matière solide est produit.

### Lavage du flux de matière solide :

Afin de récupérer les sucres piégés dans la matière solide, une étape de lavage de la matière solide est mise en oeuvre. Dans cet exemple, la totalité de l'eau envoyée comme eau de dilution à l'étape d'hydrolyse enzymatique dans l'unité H provient des vinasses produites en distillation dans l'unité S. Ces vinasses sont d'abord utilisées pour laver la matière solide dans l'unité de lavage L à contrecourant sous trois étages de contact. Puis les vinasses issues de l'unité L sont introduites dans l'unité H. **Les pertes d'hexoses sont alors réduites de 92,0% par rapport aux résultats de l'exemple 5, soit 1,6% de perte globale. Les vinasses étant plus concentrées en pentoses qu'en hexoses, les pertes globales en pentoses observées sont de l'ordre de 19,1%, 7,7% inférieures aux pertes de l'exemple 5.**

### Conversion en éthanol :

Le liquide clarifié est envoyé vers les fermenteurs de l'unité F. Le microorganisme de fermentation est *Saccharomyces cerevisiae* provenant d'une souche modifiée capable de réaliser la co-fermentation des hexoses et des pentoses. Couplé aux rendements de l'hydrolyse enzymatique, le rendement global de la conversion de la cellulose en éthanol est de 0,48 g d'éthanol par g de cellulose introduite. La conversion des pentoses en éthanol s'élève à 0,09 g d'éthanol par g de pentose introduite. La baisse de rendement s'explique par la plus haute concentration de produits hydrolysés dans l'étape d'hydrolyse enzymatique. Le titre éthanolique du flux envoyé en distillation est de 65,8 g d'éthanol / kg de vin.

### Séparation et récupération de l'éthanol :

La séparation se fait par distillation dans l'unité S. Le rendement d'extraction est de 99,6%. Ainsi, le procédé permet de produire annuellement **112 379 tonnes d'éthanol,** et a une consommation spécifique d'eau de procédé de **0,0 tonne d'eau** / **tonne d'éthanol produit en régime continu.**

## Revendications

1. Procédé de production d'alcool et/ou de solvant à partir d'une charge de biomasse, dans lequel on effectue les étapes suivantes :
a) on effectue une étape de prétraitement par mise en contact et chauffage de la charge de biomasse avec de l'eau et un composé acide ou basique, de manière à obtenir un substrat prétraité,
b) on met en contact le substrat prétraité avec au moins des enzymes cellulases et avec un flux liquide de lavage obtenu à l'étape d) de manière à obtenir un hydrolysat comportant une matière solide et une phase liquide contenant des sucres,
c) on extrait au moins une partie de la matière solide contenue dans l'hydrolysat de manière à obtenir un hydrolysat appauvri en matière solide et un flux enrichi en matière solide,
d) on lave le flux enrichi en matière solide avec un flux liquide comportant au moins une partie du flux de vinasse obtenu à l'étape f) de manière à obtenir ledit flux liquide de lavage, le flux liquide de lavage étant recyclé à l'étape b),
e) on effectue une fermentation alcoolique de l'hydrolysat appauvri en matière solide obtenu à l'étape c) au moyen d'un microorganisme alcooligène de manière à produire un vin de fermentation,
f) on effectue une étape de séparation du vin de fermentation de manière à obtenir au moins un flux purifié comportant un alcool ou un solvant et au moins un flux de vinasse.

2. Procédé selon la revendication 1, dans lequel à l'étape b), le flux liquide de lavage a un débit compris entre 50% et 1500% poids du débit de substrat prétraité.

3. Procédé selon l'une des revendications 1 et 2, dans lequel avant d'effectuer l'étape b), le flux liquide de lavage obtenu à l'étape d) est soumis à une deuxième étape de fermentation alcoolique au moyen d'un organisme alcooligène.

4. Procédé selon la revendication 3, dans lequel on effectue la deuxième étape de fermentation alcoolique dans des conditions opératoires différentes des conditions opératoires de la fermentation alcoolique de l'étape e).

5. Procédé selon l'une des revendications 3 et 4, dans lequel la deuxième étape de fermentation alcoolique avec un organisme alcooligène différent de l'organisme alcooligène de la fermentation alcoolique de l'étape a).

6. Procédé selon l'une des revendications précédentes, dans lequel à l'étape d), on met en contact le flux enrichi en matière solide avec un flux liquide, puis on sépare le flux liquide de la matière solide.

7. Procédé selon l'une des revendications précédentes, dans lequel on réalise l'étape d) de manière à ce que ledit flux enrichi en matière solide comporte entre 15% poids et 55% poids de matière solide et de manière à ce que ledit hydrolysat appauvri en matière solide comporte moins de 15% poids de matière solide.

8. Procédé selon l'une des revendications précédentes, dans lequel les enzymes cellulases et/ou hemicellulases sont produites par un microorganisme choisi parmi les champignons appartenant aux genres *Trichoderma, Aspergillus, Penicillium ou Schizophyllum,* ou les bactéries anaérobies appartenant au genre *Clostridium.*

9. Procédé selon l'une des revendications précédentes, dans lequel le microorganisme alcooligène est choisi parmi les microorganismes du genre *Saccharomyces, Saccharomyces cerevisiae, Schizosaccharomyces pombe, Saccharomyces uvarum, Saccharomyces diastaticus, Kluyveromyces fragilis, Candida shehatae, Pichia stipitis, Pachysolen tannophilis*, *Zymomonas mobilis, Clostridium, Escherichia coli.*

10. Procédé selon l'une des revendications précédentes, dans lequel la charge de biomasse est composée par au moins l'un des éléments suivants : du bois, de plantes cultivées, des déchets lignocellulosiques agricoles, des résidus de l'industrie de transformation des matériaux lignocellulosiques.

11. Procédé selon l'une des revendications précédentes, dans lequel à l'étape a), on effectue une explosion à la vapeur de la biomasse en comprimant, puis en effectuant une détente de la biomasse en mélange avec de l'eau et un composé acide.

12. Procédé selon l'une des revendications précédentes, dans lequel à l'étape e) on met en oeuvre un organisme alcooligène produisant au moins de l'éthanol.

## Patentansprüche

1. Verfahren zur Herstellung von Alkohol oder Lösungsmittel aus einer Biomassecharge, wobei die folgenden Schritte durchgeführt werden:
a) ein Schritt der Vorbehandlung durch Inkontaktbringen und Erhitzen der Biomassecharge mit Wasser und einer sauren oder basischen Verbindung wird durchgeführt, um ein vorbehandeltes Substrat zu erhalten,
b) das vorbehandelte Substrat wird mit mindestens Cellulaseenzymen und mit einem Waschflüssigkeitsstrom in Kontakt gebracht, der in Schritt d) erhalten wird, um ein Hydrolysat zu erhalten, das einen Feststoff und eine Flüssigphase umfasst, die Zucker enthält,
c) mindestens ein Teil des Feststoffs wird extrahiert, der im Hydrolysat enthalten ist, um ein an Feststoff verarmtes Hydrolysat und einen mit Feststoff angereicherten Strom zu erhalten,
d) der mit Feststoff angereicherte Strom wird mit einem Flüssigkeitsstrom gewaschen, der mindestens einen Teil des in Schritt f) erhaltenen Schlempenstroms umfasst, um den Waschflüssigkeitsstrom zu erhalten, wobei der Waschflüssigkeitsstrom zu Schritt b) rückgeführt wird,
e) eine Alkoholfermentation des an Feststoff verarmten Hydrolysats, das in Schritt c) erhalten wird, wird mit einem alkoholerzeugenden Mikroorganismus durchgeführt, um einen Fermentationswein zu erzeugen,
f) ein Trennschritt des Fermentationsweins wird durchgeführt, um mindestens einen gereinigten Strom zu erhalten, der einen Alkohol oder ein Lösungsmittel und mindestens einen Schlempenstrom umfasst.

2. Verfahren nach Anspruch 1, wobei in Schritt b) der Waschflüssigkeitsstrom einen Durchsatz zwischen 50 Gew.-% und 1500 Gew.-% des Durchsatzes des vorbehandelten Substrats aufweist.

3. Verfahren nach einem der Ansprüche 1 und 2, wobei vor der Durchführung von Schritt b) der in Schritt d) erhaltene Waschflüssigkeitsstrom einem zweiten Alkoholfermentationsschritt mit einem alkoholerzeugenden Mikroorganismus unterzogen wird.

4. Verfahren nach Anspruch 3, wobei der zweite Alkoholfermentationsschritt unter anderen Betriebsbedingungen durchgeführt wird als den Betriebsbedingungen der Alkoholfermentation in Schritt e) .

5. Verfahren nach einem der Ansprüche 3 oder 4, wobei der zweite Alkoholfermentationsschritt mit einem anderen alkoholerzeugenden Mikroorganismus durchgeführt wird als dem alkoholerzeugenden Mikroorganismus der Alkoholfermentation von Schritt a).

6. Verfahren nach einem der vorhergehenden Ansprüche, wobei in Schritt d) der mit Feststoff angereicherte Strom mit einem Flüssigkeitsstrom in Kontakt gebracht wird, dann der Flüssigkeitsstrom von dem Feststoff getrennt wird.

7. Verfahren nach einem der vorhergehenden Ansprüche, wobei der Schritt d) derart durchgeführt wird, dass der mit Feststoff angereicherte Strom zwischen 15 Gew.-% und 55 Gew.-% Feststoff umfasst, und derart, dass das an Feststoff verarmte Hydrolysat weniger als 15 Gew.-% Feststoff umfasst.

8. Verfahren nach einem der vorhergehenden Ansprüche, wobei die Cellulase- und/oder Hemicellulaseenzyme von einem Mikroorganismus erzeugt werden, ausgewählt aus Pilzen, die zu den Gattungen *Trichoderma, Aspergillus, Penicillium oder Schizophyllium* gehören, oder anaeroben Bakterien, die zur Gattung *Clostridium* gehören.

9. Verfahren nach einem der vorhergehenden Ansprüche, wobei der alkoholerzeugende Mikroorganismus ausgewählt wird aus Mikroorganismen der Gattung *Saccharomyces, Saccharomyces cerevisiae, Schizosaccharomyces pombe, Saccharomyces uvarum, Saccharomyces diastaticus, Kluyveromyces fragilis, Candida shehatae, Pichia stipitis, Pachysolen tannophilis, Zymomonas mobilis, Clostridium, Escherichia coli.*

10. Verfahren nach einem der vorhergehenden Ansprüche, wobei die Biomassecharge aus mindestens einem der folgenden Elemente besteht: Holz, Kulturpflanzen, landwirtschaftlichen Lignocelluloseabfällen, Industrierückständen der Transformation von Lignocellulosematerialien.

11. Verfahren nach einem der vorhergehenden Ansprüche, wobei in Schritt a) eine Dampfexplosion der Biomasse durch Kompression durchgeführt wird, dann mittels der Durchführung einer Entspannung der Biomasse in Mischung mit dem Wasser und einer sauren Verbindung.

12. Verfahren nach einem der vorhergehenden Ansprüche, wobei in Schritt e) ein alkoholerzeugender Organismus verwendet wird, der mindestens Ethanol erzeugt.

## Claims

1. Process for the production of alcohol and/or solvent from a biomass feedstock, in which the following stages are carried out:
a) A pretreatment stage is carried out by heating and bringing into contact the biomass feedstock with water and an acid or basic compound in such a way as to obtain a pretreated substrate,
b) The pretreated substrate is brought into contact with at least cellulase enzymes and with a liquid washing stream obtained in stage d) in such a way as to obtain a hydrolyzate that comprises a solid material and a liquid phase containing sugars,
c) At least a portion of the solid material contained in the hydrolyzate is extracted in such a way as to obtain a hydrolyzate that is low in solid material and a stream that is enriched with solid material,
d) The stream that is enriched with solid material is washed with a liquid stream comprising at least a portion of the vinasse stream obtained in step f) in such a way as to obtain said liquid washing stream, with the liquid washing stream being recycled in stage b),
e) An alcoholic fermentation of the hydrolyzate that is low in solid material obtained in stage c) is carried out by means of an alcohologenic microorganism in such a way as to produce a fermentation wine,
f) A stage for separation of the fermentation wine is carried out in such a way as to obtain at least a purified stream comprising an alcohol or a solvent and at least one vinasse stream.

2. Process according to Claim 1, in which in stage b), the liquid washing stream has a flow rate of between 50% and 1500% by weight of the flow rate of pretreated substrate.

3. Process according to one of Claims 1 and 2, in which before carrying out stage b), the liquid washing stream obtained in stage d) is subjected to a second stage for alcoholic fermentation by means of an alcohologenic organism.

4. Process according to Claim 3, in which the second stage of alcoholic fermentation is carried out under operating conditions that are different from the operating conditions of the alcoholic fermentation of stage e).

5. Process according to one of Claims 3 and 4, in which the second stage of alcoholic fermentation is carried out with an alcohologenic organism that is different from the alcohologenic organism of the alcoholic fermentation of stage e).

6. Process according to one of the preceding claims, in which in stage d), the stream that is enriched with solid material is brought into contact with a liquid stream, and then the liquid stream is separated from the solid material.

7. Process according to one of the preceding claims, in which stage c) is carried out in such a way that said stream that is enriched with solid material comprises between 15% by weight and 55% by weight of solid material and in such a way that said hydrolyzate that is low in solid material comprises less than 15% by weight of solid material.

8. Process according to one of the preceding claims, in which the cellulase enzymes are produced by a microorganism that is selected from among the mushrooms that belong to the genera *Trichoderma, Aspergillus, Penicillium,* or *Schizophyllum,* or the anaerobic bacteria that belong to the genus *Clostridium.*

9. Process according to one of the preceding claims, in which the alcohologenic microorganism is selected from among the microorganisms of the genus *Saccharomyces, Saccharomyces cerevisiae, Schizosaccharomyces pombe, Saccharomyces uvarum, Saccharomyces diastaticus, Kluyveromyces fragilis, Candida shehatae, Pichia stipitis, Pachysolen tannophilis, Zymomonas mobilis, Clostridium, Escherichia coli.*

10. Process according to one of the preceding claims, in which the biomass feedstock consists of at least one of the following elements: wood, cultivated plants, agricultural lignocellulosic waste, residues of the industry for transformation of the lignocellulosic materials.

11. Process according to one of the preceding claims, in which in stage a), a vapor explosion of the biomass is carried out by exerting compression and then by carrying out pressure relief of the biomass mixed with water and an acid compound.

12. Process according to one of the preceding claims, in which in stage e), an alcohologenic organism that produces at least ethanol is used.
